# EUROPEAN PATENT SPECIFICATION

(11) **EP 1 249 452 B1**
(45) Date of publication and mention of the grant of the patent: **30.06.2004**
(21) Application number: 01108841.6
(22) Date of filing: 09.04.2001
(51) Int. Cl.: C07D 487/04, A01N 43/90

(54) **Fungicidal 5-Alkylamino-6-phenyl-7-halo-triazolopyrimidines**
Fungizide 5-Alkylamino-6-phenyl-7-halogen-Triazolopyrimidine
5-Alkylamino-6-phényle-7-halogène triazolopyrimidine fongicides

(43) Date of publication of application: 16.10.2002
(73) Proprietor: BASF AKTIENGESELLSCHAFT, 67056 Ludwigshafen (DE)
(72) Inventor: Tormo i Blasco, Jordi, Dr., 67117 Limburgerhof (DE); Ammermann, Eberhard, Dr., 64646 Heppenheim (DE); Pees, Klaus-Jürgen, Dr., 55192 Mainz (DE); Pfrengle, Waldemar, Dr., 55444 Seibersbach (DE)

(56) References cited:
- EP-A- 0 071 792
- EP-A- 0 550 113
- EP-A- 0 782 997
- WO-A-94/20501
- WO-A-98/46607
- WO-A-99/41255

## Description

The invention relates to 5-alkylamino-6-phenyl-7-halo-triazolopyrimidines of formula I 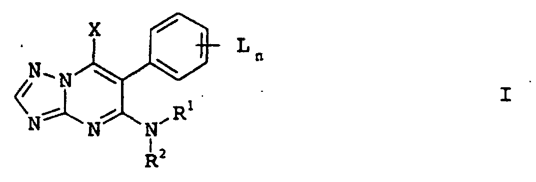 in which
- R¹ and R²: independently denote hydrogen or
C₁-C₁₀-alkyl, C₂-C₁₀-alkenyl, C₂-C₁₀-alkynyl, C₄-C₁₀-alkadienyl, or C₁-C₁₀-haloalkyl,
C₃-C₈-cycloalkyl, C₅-C₁₀-bicycloalkyl, phenyl, naphthyl, or 5- or 6-membered heterocyclyl, containing one to four nitrogen atoms or one to three nitrogen atoms and one sulfur or oxygen atom, or
5- or 6-membered heteroaryl, containing one to four nitrogen atoms or one to three nitrogen atoms and one sulfur or oxygen atom, or
where R¹ and R² radicals may be unsubstituted or partially or fully halogenated or may carry one to three groups R^{a},
R^{a} is halogen, cyano, nitro, hydroxyl, C₁-C₆-alkyl, C₁-C₆-haloalkyl, C₃-C₆-cycloalkyl, C₁-C₆-alkoxy, C₁-C₆-haloalkoxy, C₁-C₆-alkylthio, C₁-C₆-alkylamino, di-C₁-C₆-alkylamino, C₂-C₆-alkenyl, C₂-C₆-alkenyloxy, C₂-C₆-alkynyl, C₃-C₆-alkynyloxy and C₁-C₄-alkylenedioxy, which may be halogenated; or
- R¹ and R²: together with the interjacent nitrogen atom represent a 5- or 6-membered heterocyclic ring, containing one to four nitrogen atoms or one to three nitrogen atoms and one sulfur or oxygen atom, which may be substituted by one to three R^{a} radicals;
- L: is hydrogen, halogen, C₁-C₁₀-alkyl, C₁-C₁₀-alkoxy and C₁-C₁₀-haloalkyl;
- n: is an integer from 1 to 5; and
- X: is halogen.

Moreover, the invention relates to processes for their preparation, compositions containing them and to their use for combating phytopathogenic fungi.

EP-A 071 792 discloses 6-phenyl-7-amino-triazolopyrimidines where the 5-position is substituted by hydrogen or alkyl or aryl groups.

EP-A 550 113 relates to 5-H- and 5-halogen-6-phenyl-7-amino-triazolopyrimidines where the 7-amino group is further substituted.

WO-A 98/46607 discloses funcidal triazolopyrimidines, which are substituted in the 6-position by a 2,4,6-trifluorophenyl group.

The compounds disclosed in the documents discussed above are said to be active against various phytopathogenic fungi.

It is an object of the present invention to provide compounds having improved fungicidal activity.

We have found that this object is achieved by the compounds defined at the outset. Furthermore, we have found processes for their preparation, compositions comprising them and methods for controlling phytopathogenic fungi using the compounds I.

The compounds of the formula I differ from the compounds known from the abovementioned art in the specific combination of the 5-alkylamino, 6-phenyl group and the halogen atom amino group in 5-position of the triazolopyrimidine system.

A 4- to 6- membered heterocyclic group may be any heterocyclic group with 4 to 6 ring atoms, interrupted by one or more heteroatoms selected from sulfur, nitrogen, and oxygen, preferably oxygen. A halogen atom suitable denotes a fluorine, chlorine or bromine atom.

The present invention further provides a process for the preparation of compounds of formula I as defined above which comprises treating a 5,7-dihalo compound of formula II in which X is halogen with a hydroxide to 7-hydroxycompounds of formula III

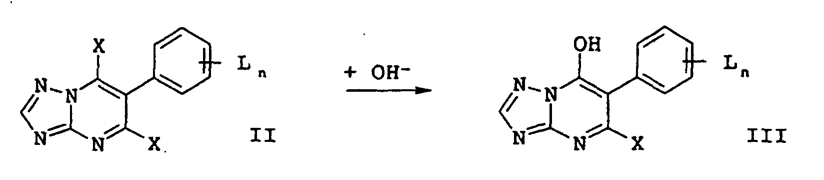

The reaction of compounds II with hydroxide is usually carried out in water and organic solvent at from 0°C to 80°C, preferably at from 20°C to 60°C.

Suitable solvents are ethers, such as tetrahydrofuran and alcohols. It is also possible to use mixtures of the solvents mentioned.

Suitable hydroxides are, in general, inorganic compounds, such as alkali metal and alkaline earth metal hydroxides (e.g. lithium hydroxide, sodium hydroxide, potassium hydroxide and calcium hydroxide).

It may be advantageous for the reaction to add a catalytic amount of a crown ether (e.g. 18-crown-6 or 15-crown-5).

The reaction can also be carried out in two-phase systems comprising a solution of alkali metal or alkaline earth metal hydroxides or carbonates in water and in organic phase (e.g. aromatic and/or halogenated hydrocarbons). Suitable phase transfer catalysts are, for example, ammonium halides and ammonium tetrafluoroborates (e.g. benzyltriethylammonium chloride, benzyltributylammonium bromide, tetrabutyl ammonium chloride, hexadecyl trimethyl ammonium bromide or tetrabutylammonium tetrafluoroborate) and phosphonium halides (e.g. tetrabutylphosphonium chloride and tetraphenylphosphonium bromide).

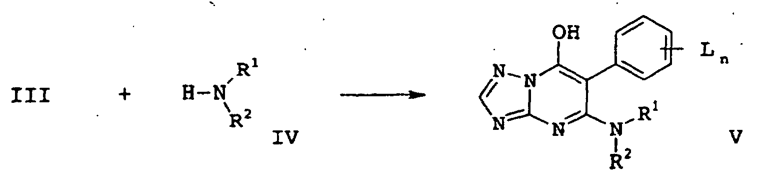

The reaction between the 5-halo-6-phenyl compound III and the amine of formula IV is preferably carried out in the presence of a solvent.

Suitable solvents include ethers, such as dioxane, diethyl ether and, especially, tetrahydrofuran, halogenated hydrocarbons such as dichloromethane and aromatic hydrocarbons, for example toluene.

The reaction is suitably carried out at a temperature in the range from 0°C to 70°C, the preferred reaction temperature being from 10°C to 35°C.

It is also preferred that the reaction is carried out in the presence of a base. Suitable bases include tertiary amines, such as triethylamine, and inorganic bases, such as potassium carbonate. or sodium carbonate. Alternatively, an excess of the compound of formula V may serve as a base.

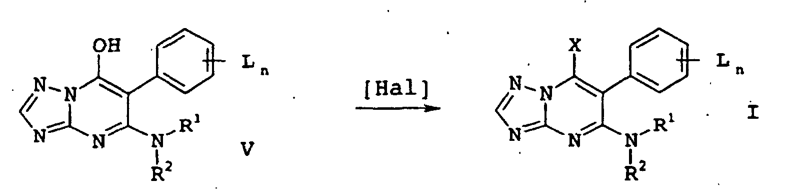

Compound of formula V is subsequently treated with a halogenating agent [Hal], preferably with a brominating or chlorinating agent, such as phosphorus oxybromide or phosphorus oxychloride, neat or in the presence of a solvent to give I.

The reaction is suitably carried out at a temperature in the range from 0 °C to 150 °C, the preferred reaction temperature being from 80 °C to 125 °C as disclosed for example by EP-A 770 615.

The reaction mixtures are worked up in a customary manner, for example by mixing with water, phase separation and, if required, chromatographic purification of the crude products. Some of the end products are obtained in the form of colorless or slightly brownish, viscous oils, which are purified or freed from volatile components under reduced pressure and at moderately elevated temperatures. If the end products are obtained as solids, purification can also be carried out by recrystallization or digestion.

Compounds of formula II are known in the art and can be obtained by synthesis routes disclosed in EP-A 550 113 and WO-A 98/46608.

If individual compounds I are not obtainable by the routes described above, they can be prepared by derivatization of other compounds I.

In the symbol definitions given in the formulae above, collective terms were used which generally represent the following substituents:
- halogen: fluorine, chlorine, bromine and iodine;
- C₁-C₁₀-alkyl and the alkyl moieties of C₁-C₁₀-haloalkyl: saturated, straight-chain or branched hydrocarbon radicals having 1 to 10, especially 1 to 6 carbon atoms, for example C₁-C₄-alkyl as mentioned above or pentyl, 1-methylbutyl, 2-methylbutyl, 3-methylbutyl, 2,2-di-methylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl; 1,2,2-trimethylpropyl, 1-ethyl-1-methylpropyl and 1-ethyl-2-methylpropyl;
- C₁-C₆-haloalkyl and the haloalkyl moieties of C₁-C₆-haloalkoxy: straight-chain or branched alkyl groups having 1 to 6, preferably 1 to 4 carbon atoms (as mentioned above), where the hydrogen atoms in these groups may be partially or fully replaced by halogen atoms as mentioned above, for example C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl;
- C₃-C₁₀-alkenyl: unsaturated, straight-chain or branched hydrocarbon radicals having 3 to 10, especially 3 to 6 carbon atoms and a double bond in any position, for example 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl and 2-methyl-2-propenyl;
C₂-C₁₀-alkynyl: straight-chain or branched hydrocarbon radicals having 2 to 10, especially 2 to 4 carbon atoms and a triple bond in any position, for example ethynyl, 1-propynyl, 2-propynyl, 1-butynyl, 2-butynyl, 3-butynyl and 1-methyl-2-propynyl;

In general terms, the term cycloalkyl, as used herein with respect to a radical or moiety refers to a cycloalkyl group having 3 to 8 carbon atoms, preferably 5 to 7 carbon atoms.

In general terms, the term bicycloalkyl, as used herein with respect to a radical or moiety refers to a bicycloalkyl group having 5 to 10 carbon atoms, preferably 6 to 9 carbon atoms, in particular bicycloheptyl being optionally substituted by one or more halogen atoms, nitro, cyano, alkyl, preferably C₁₋₆ alkyl, alkoxy, preferably C₁₋₆ alkoxy.

5-membered heteroaryl, containing one to four nitrogen atoms or one to three nitrogen atoms and one sulfur or oxygen atom: 5-membered heteroaryl groups which, in addition to carbon atoms, may contain one to four nitrogen atoms or one to three nitrogen atoms and one sulfur or oxygen atom as ring members, for example 2-furyl, 3-furyl, 2-thienyl, 3-thienyl, 2-pyrrolyl, 3-pyrrolyl, 3-isoxazolyl, 4-isoxazolyl, 5-isoxazolyl, 3-isothiazolyl, 4-isothiazolyl, 5-isothiazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,2,4-triazol-3-yl, 1,3,4-oxadiazol-2-yl, 1,3,4-thiadiazol-2-yl and 1,3,4-triazol-2-yl;

6-membered heteroaryl, containing one to four nitrogen atoms: 6-membered heteroaryl groups which, in addition to carbon atoms, may contain one to three or one to four nitrogen atoms as ring members, for example 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin-3-yl.

With respect to their intended use, preference is given to triazolopyrimidines of the formula I having the following substituents, where the preference is valid in each case on its own or in combination:

The particularly preferred embodiments of the intermediates with respect to the variables correspond to those of the radicals X, R¹ and R² of formula I.

A preferred alkyl moiety is an ethyl or especially a methyl group.

A preferred haloalkyl moiety is the 2,2,2-trifluoroethyl or 1,1,1-trifluoroprop-2-yl group;

A preferred alkenyl moiety is allyl or especially a 2-methylallyl group.

A preferred cycloalkyl moiety is cyclopentyl being optionally substituted by one or more halogen atoms, nitro, cyano, alkyl, preferably C₁-C₆ alkyl, alkoxy, preferably C₁-C₆ alkoxy.

A preferred heteroaryl moiety is pyridyl, pyrimidyl, pyrazolyl or thienyl.

Preference is given to compounds of formula I in which any alkyl or haloalkyl part of the groups R¹ or R², which may be straight chained or branched, contains up to 10 carbon atoms, preferably 1 to 9 carbon atoms, more preferably 2 to 6 carbon atoms, any alkenyl or alkynyl part of the substituents R¹ or R² contains up to 10 carbon atoms, preferably 2 to 9 carbon atoms, more preferably 3 to 6 carbon atoms, any cycloalkyl part of the substituents R¹ or R² contains from 3 to 10 carbon atoms, preferably from 3 to 8 carbon atoms, more preferably from 3 to 6 carbon atoms, and any bicycloalkyl part of the substituents R¹ or R² contains from 5 to 9 carbon atoms, preferably from 7 to 9 carbon atoms. Any alkyl, alkenyl or alkynyl group may be linear or branched.

Likewise, preference is given to compounds of formula I wherein R¹ is not hydrogen.

Compounds of formula I are preferred in which R¹ represents a straight-chained or branched C₁-C₁₀-alkyl, in particular a branched C₃-C₁₀-alkyl group, a C₃-C₈-cycloalkyl, a C₅-C₉-bicycloalkyl, a C₃-C₈-cycloalkyl-C₁-C₆-alkyl, C₁-C₁₀-alkoxy-C₁-C₆-alkyl, a C₁-C₁₀-haloalkyl or a phenyl group being optionally substituted by one to three halogen atoms or C₁-C₁₀-alkyl or C₁-C₁₀-alkoxy groups.

Particular preference is given to compounds I in which R² represents a hydrogen atom, a C₁-C₁₀-alkyl or a C₁-C₁₀-haloalkyl group, in particular a hydrogen atom.

Besides, particular preference is given to compounds I in which R² is hydrogen.

Moreover, particular preference is given to compounds I in which R² is methyl.

Furthermore, particular preference is given to compounds I in which R² is ethyl.

If R¹ denotes a C₁-C₁₀-haloalkyl group, preferably a polyfluorinated alkyl group, in particular a 2,2,2-trifluoroethyl, a 2-(1,1,1-trifluoropropyl) or a 2-(1,1,1-trifluorobutyl) group, R² preferably represents a hydrogen atom.

If R¹ denotes an optionally substituted C₃-C₈-cycloalkyl group, preferably a cyclopentyl or cyclohexyl group, R² preferably represents a hydrogen atom or C₁-C₆-alkyl group.

Moreover, particular preference is given to compounds I in which R¹ and R² together with the interjacent nitrogen atom form an optionally substituted heterocyclic ring, preferably an optionally substituted C₃-C₇-heterocyclic ring, in particular a pyrrolidine, piperidine, tetrahydropyridine, in particular 1,2,3,6-tetrahydropyridine or azepane ring which is optionally substituted by one or more C₁-C₁₀-alkyl groups.

Lₙ preferably is halogen or C₁-C₆-alkoxy. A preferred embodiment are compounds of formula I in which 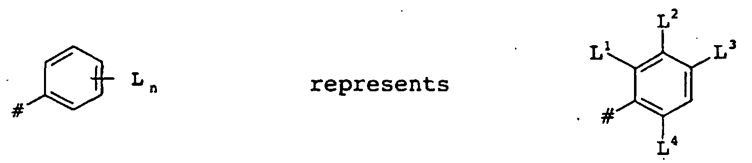 wherein # denotes the link to the triazolopyrimidine moiety, L¹ through L⁴ each independently represent hydrogen, especially fluorine, chlorine, methyl or methoxy, in particular wherein L¹ is fluoro, L² is hydrogen or fluoro, L³ is hydrogen or fluoro or methoxy and L⁴ denotes hydrogen, fluoro chloro or methyl.

Moreover, particular preference is given to compounds of the formula I in which n is 2 or 3. Most preferred L⁴ is not hydrogen.

Furthermore, particular preference is given to compounds of formula IA in which the variables have the meaninng as defined in formula I.

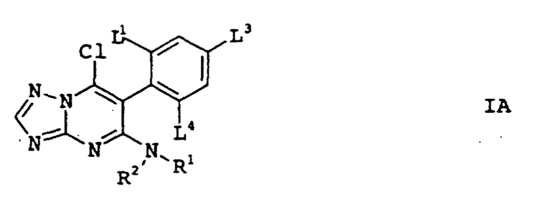

Preference is given to compounds of formula IA in which L¹ denotes halogen and L³ and L⁴ each independently represent hydrogen, halogen or C₁-C₄-alkoxy.

Most preferred are compounds of formula IA in which L¹ is fluoro, L³ is hydrogen and L⁴ is chloro.

Included in the scope of the present Invention are (R) and (S) isomers of compounds of general formula I having a chiral center and the racemates thereof, and salts, N-Oxides and acid addition compounds.

Particularly preference is given to compounds of formula IA wherein R² is hydrogen, L¹ and L⁴ independently represent fluorine or chlorine atoms, and L³ denotes hydrogen, fluorine, chlorine or methoxy.

Included in the scope of the present invention are (R) and (S) isomers of compounds of general formula I having a chiral center and the racemates thereof, and salts, N-oxides and acid addition compounds.

With respect to their use, particular preference is given to the compounds I compiled in the tables below. The groups mentioned in the table for a substituent are furthermore for their part, independently of the combination in which they are mentioned, a particularly preferred embodiment of the respective substituents.
Table 1
   Compounds of the formula IA, in which L¹ is fluoro, L³ is hydrogen, L⁴ is chloro and R¹ and R² correspond to one row in Table A
Table 2
   Compounds of the formula IA, in which L¹ and L⁴ each are fluoro, L³ is hydrogen and R¹ and R² correspond to one row in Table A
Table 3
   Compounds of the formula IA, in which L¹ and L⁴ each are chloro, L³ is hydrogen and R¹ and R² correspond to one row in Table A
Table 4
   Compounds of the formula IA, in which L¹ is methyl, L³ is hydrogen, L⁴ is fluoro and R¹ and R² correspond to one row in Table A
Table 5
   Compounds of the formula IA, in which L¹, L³ and L⁴ each are fluoro and R¹ and R² correspond to one row in Table A
Table 6
   Compounds of the formula IA, in which L¹ and L⁴ each are fluoro, L³ is methoxy and R¹ and R² correspond to one row in Table A

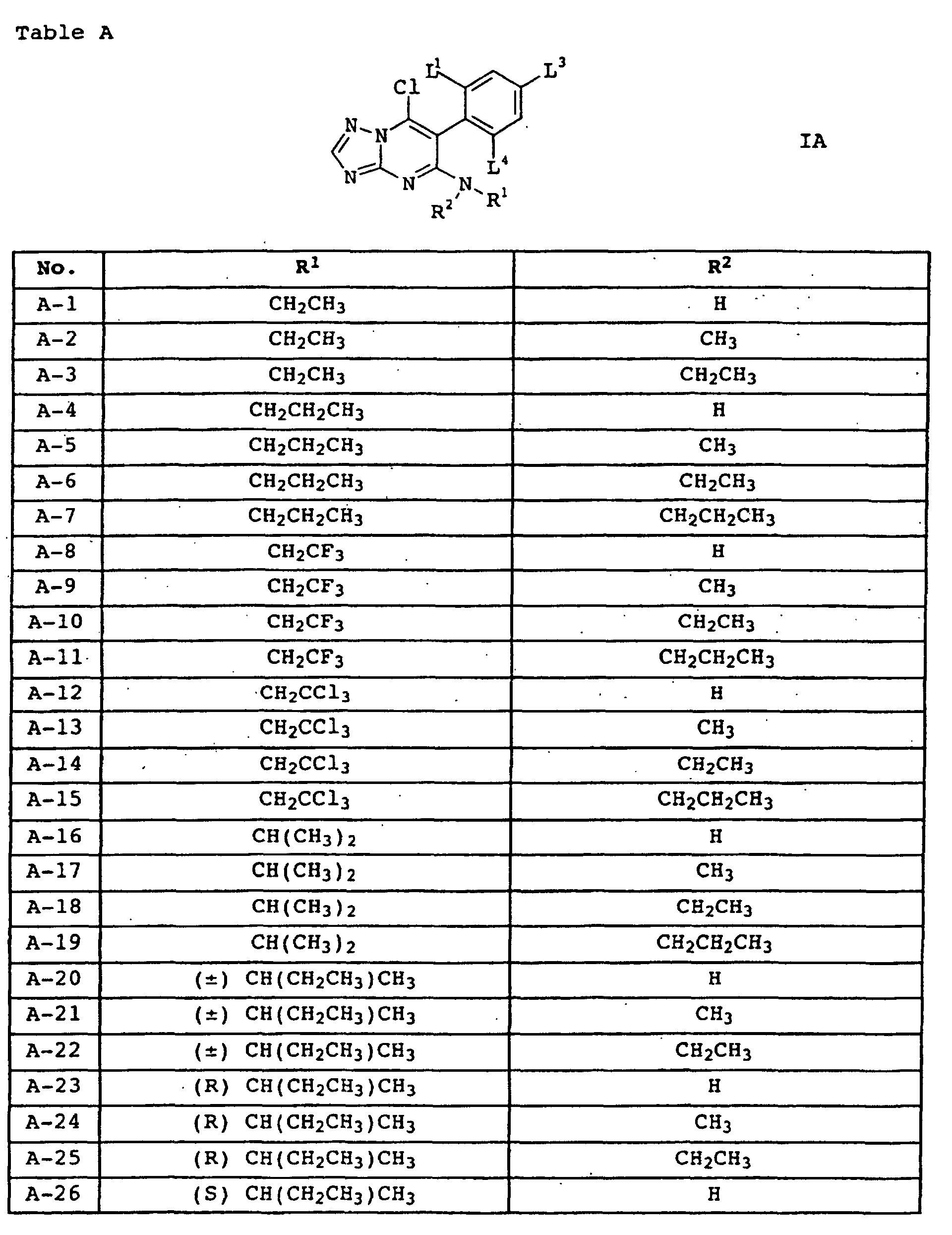

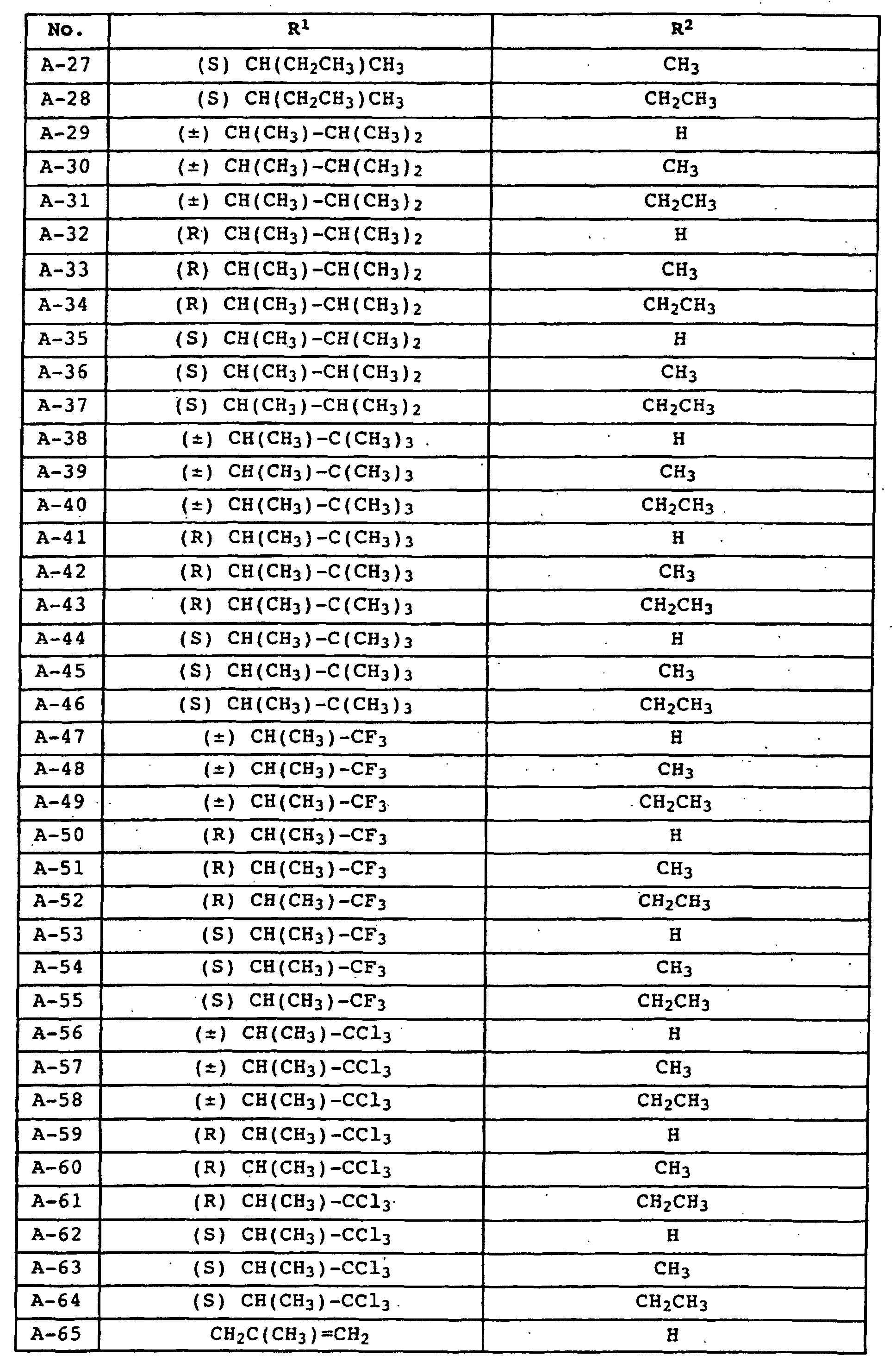

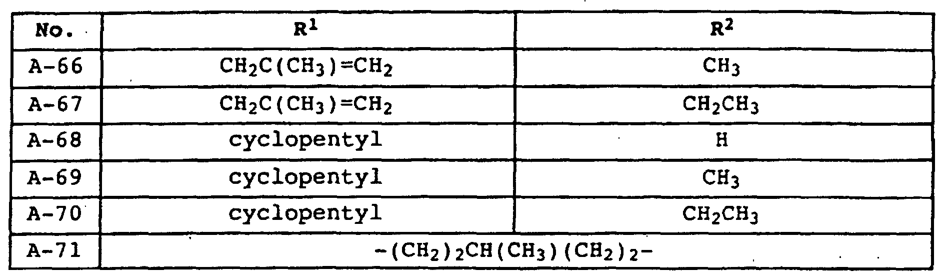

The compounds I are suitable as fungicides. They have outstanding activity against a broad spectrum of phytopathogenic fungi, in particular from the classes of the *Ascomycetes, Deuteromycetes*, *Phycomycetes* and *Basidiomycetes*. Some of them act systemically, and they can be employed in crop protection as foliar- and soil-acting fungicides.

They are especially important for controlling a large number of fungi on a variety of crop plants such as wheat, rye, barley, oats, rice, maize, grass, bananas, cotton, soya, coffee, sugar cane, grapevines, fruit species, ornamentals and vegetables such as cucumbers, beans, tomatoes, potatoes and cucurbits, and on the seeds of these plants.

Specifically, they are suitable for controlling the following plant diseases:
Alternaria species on vegetables and fruit,
Botrytis cinerea (gray mold) on strawberries, vegetables, ornamentals and grapevines,
Cercospora arachidicola on peanuts,
Erysiphe cichoracearum and Sphaerotheca fuliginea on cucurbits,
Erysiphe graminis (powdery mildew) on cereals,
Fusarium and Verticillium species on various plants,
Helminthosporium species on cereals,
Mycosphaerella species on bananas and peanuts,
Phytophthora infestans on potatoes and tomatoes,
Plasmopara viticola on grapevines,
Podosphaera leucotricha on apples,
Pseudocercosporella herpotrichoides on wheat and barley,
Pseudoperonospora species on hops and cucumbers,
Puccinia species on cereals,
Pyricularia oryzae on rice,
Rhizoctonia species on cotton, rice and lawns,
Septoria nodorum on wheat,
Uncinula necator on grapevines,
Ustilago species on cereals and sugar cane, and
Venturia species (scab) on apples and pears.

Moreover, the compounds I are suitable for controlling harmful fungi such as Paecilomyces variotii in the protection of materials (e.g. wood, paper, paint dispersions, fibers and tissues) and in the protection of stored products.

The compounds I are applied by treating the fungi, or the plants, seeds, materials or the soil to be protected against fungal infection, with a fungicidally active amount of the active ingredients. Application can be effected both before and after infection of the materials, plants or seeds by the fungi.

In general, the fungicidal compositions comprise from 0.1 to 95, preferably 0.5 to 90, % by weight of active ingredient.

When used in crop protection, the rates of application are from 0.01 to 2.0 kg of active ingredient per ha, depending on the nature of the effect desired.

In the treatment of seed, amounts of active ingredient of from 0.001 to 0.1 g, preferably 0.01 to 0.05 g, are generally required per kilogram of seed.

When used in the protection of materials or stored products, the rate of application of active ingredient depends on the nature of the field of application and on the effect desired. Rates of application conventionally used in the protection of materials are, for example, from 0.001 g to 2 kg, preferably 0.005 g to 1 kg, of active ingredient per cubic meter of material treated.

The compounds I can be converted into the customary formulations, e.g. solutions, emulsions, suspensions, dusts, powders, pastes and granules. The use form depends on the particular purpose; in any case, it should guarantee a fine and uniform distribution of the compound according to the invention.

The formulations are prepared in a known manner, e.g. by extending the active ingredient with solvents and/or carriers, if desired using emulsifiers and dispersants, it also being possible to use other organic solvents as auxiliary solvents if water is used as the diluent. Auxiliaries which are suitable are essentially: solvents such as aromatics (e.g. xylene), chlorinated aromatics (e.g. chlorobenzenes), paraffins (e.g. mineral oil fractions), alcohols (e.g. methanol, butanol), ketones (e.g. cyclohexanone), amines (e.g. ethanolamine, dimethylformamide) and water; carriers such as ground natural minerals (e.g. kaolins, clays, talc, chalk) and ground synthetic minerals (e.g. highly-disperse silica, silicates); emulsifiers such as non-ionic and anionic emulsifiers (e.g. polyoxyethylene fatty alcohol ethers, alkylsulfonates and arylsulfonates) and dispersants such as lignin-sulfite waste liquors and methylcellulose.

Suitable surfactants are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates and fatty acids and their alkali metal and alkaline earth metal salts, salts of sulfated fatty alcohol glycol ether, condensates of sulfonated naphthalene and naphthalene derivatives with formaldehyde, condensates of naphthalene or of napthalenesulfonic acid with phenol or formaldehyde, polyoxyethylene octylphenyl ether, ethoxylated isooctylphenol, octylphenol, nonylphenol, alkylphenol polyglycol ethers, tributylphenyl polyglycol ethers, alkylaryl polyether alcohols, isotridecyl alcohol, fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignin-sulfite waste liquors and methylcellulose.

Substances which are suitable for the preparation of directly sprayable solutions, emulsions, pastes or oil dispersions are mineral oil fractions of medium to high boiling point, such as kerosene or diesel oil, furthermore coal 'tar oils and oils of vegetable or animal origin, aliphatic, cyclic and aromatic hydrocarbons, e.g. benzene, toluene, xylene, paraffin, tetrahydronaphthalene, alkylated naphthalenes or their derivatives, methanol, ethanol, propanol, butanol, chloroform, carbon tetrachloride, cyclohexanol, cyclohexanone, chlorobenzene, isophorone, strongly polar solvents, e.g. dimethylformamide, dimethyl sulfoxide, N-methylpyrrolidone and water.

Powders, materials for scattering and dusts can be prepared by mixing or concomitantly grinding the active substances with a solid carrier.

Granules, e.g. coated granules, impregnated granules and homogeneous granules, can be prepared by binding the active ingredients to solid carriers. Examples of solid carriers are mineral earths, such as silicas, silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, e.g. ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise of from 0.01 to 95% by weight, preferably from 0.1 to 90% by weight, of the active ingredient. The active ingredients are employed in a purity of from 90% to 100%, preferably 95% to 100% (according to NMR spectrum).

The following are exemplary formulations:
I. 5 parts by weight of a compound according to the invention are mixed intimately with 95 parts by weight of finely divided kaolin. This gives a dust which comprises 5% by weight of the active ingredient.
II. 30 parts by weight of a compound according to the invention are mixed intimately with a mixture of 92 parts by weight of pulverulent silica gel and 8 parts by weight of paraffin oil which had been sprayed onto the surface of this silica gel. This gives a formulation of the active ingredient with good adhesion properties (comprises 23% by weight of active ingredient).
III. 10 parts by weight of a compound according to the invention are dissolved in a mixture composed of 90 parts by weight of xylene, 6 parts by weight of the adduct of 8 to 10 mol of ethylene oxide and 1 mol of oleic acid N-monoethanolamide, 2 parts by weight of calcium dodecylbenzenesulfonate and 2 parts by weight of the adduct of 40 mol of ethylene oxide and 1 mol of castor oil (comprises 9% by weight of active ingredient).
IV. 20 parts by weight of a compound according to the invention are dissolved in a mixture composed of 60 parts by weight of cyclohexanone, 30 parts by weight of isobutanol, 5 parts by weight of the adduct of 7 mol of ethylene oxide and 1 mol of isooctylphenol and 5 parts by weight of the adduct of 40 mol of ethylene oxide and 1 mol of castor oil (comprises 16% by weight of active ingredient).
V. 80 parts by weight of a compound according to the invention are mixed thoroughly with 3 parts by weight of sodium diisobutylnaphthalene-alpha-sulfonate, 10 parts by weight of the sodium salt of a lignosulfonic acid from a sulfite waste liquor and 7 parts by weight of pulverulent silica gel, and the mixture is ground in a hammer mill (comprises 80% by weight of active ingredient).
VI. 90 parts by weight of a compound according to the invention are mixed with 10 parts by weight of N-methyl-α-pyrrolidone, which gives a solution which is suitable for use in the form of microdrops (comprises 90% by weight of active ingredient).
VII. 20 parts by weight of a compound according to the invention are dissolved in a mixture composed of 40 parts by weight of cyclohexanone, 30 parts by weight of isobutanol, 20 parts by weight of the adduct of 7 mol of ethylene oxide and 1 mol of isooctylphenol and 10 parts by weight of the adduct of 40 mol of ethylene oxide and 1 mol of castor oil. Pouring the solution into 100,000 parts by weight of water and finely distributing it therein gives an aqueous dispersion which comprises 0.02% by weight of the active ingredient.
VIII. 20 parts by weight of a compound according to the invention are mixed thoroughly with 3 parts by weight of sodium diisobutylnaphthalene-α-sulfonate, 17 parts by weight of the sodium salt of a lignosulfonic acid from a sulfite waste liquor and 60 parts by weight of pulverulent silica gel, and the mixture is ground in a hammer mill. Finely distributing the mixture in 20,000 parts by weight of water gives a spray mixture which comprises 0.1% by weight of the active ingredient.

The active ingredients can be used as such, in the form of their formulations or the use forms prepared therefrom, e.g. in the form of directly sprayable solutions, powders, suspensions or dispersions, emulsions, oil dispersions, pastes, dusts, materials for spreading, or granules, by means of spraying, atomizing, dusting, scattering or pouring. The use forms depend entirely on the intended purposes; in any case, this is intended to guarantee the finest possible distribution of the active ingredients according to the invention.

Aqueous use forms can be prepared from emulsion concentrates, pastes or wettable powders (sprayable powders, oil dispersions) by adding water. To prepare emulsions, pastes or oil dispersions, the substances as such or dissolved in an oil or solvent, can be homogenized in water by means of wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active ingredient concentrations in the ready-to-use products can be varied within substantial ranges. In general, they are from 0.0001 to 10%, preferably from 0.01 to 1%.

The active ingredients may also be used successfully in the ultra-low-volume process (ULV), it being possible to apply formulations comprising over 95% by weight of active ingredient, or even the active ingredient without additives.

Various types of oils, herbicides, fungicides, other pesticides, or bactericides may be added to the active ingredients, if appropriate also only immediately prior to use (tank mix). These agents can be admixed with the agents according to the invention in a weight ratio of 1:10 to 10:1.

In the use form as fungicides, the compositions according to the invention can also be present together with other active ingredients, e.g. with herbicides, insecticides, growth regulators, fungicides or else with fertilizers. Mixing the compounds I or the compositions comprising them in the use form as fungicides with other fungicides frequently results in a broader fungicidal spectrum of action.

The following list of fungicides, together with which the compounds according to the invention can be used, is intended to illustrate the possible combinations, but not to impose any limitation:
sulfur, dithiocarbamates and their derivatives, such as iron(III) dimethyldithiocarbamate, zinc dimethyldithiocarbamate, zinc ethylenebisdithiocarbamate, manganese ethylenebisdithiocarbamate, manganese zinc ethylenediaminebisdithiocarbamate, tetramethylthiuram disulfide, ammonia complex of zinc (N,N-ethylenebisdithiocarbamate), ammonia complex of zinc (N,N'-propylenebisdithiocarbamate), zinc (N,N'-propylenebisdithiocarbamate), N,N'-polypropylenebis(thiocarbamoyl)disulfide;
nitro derivatives, such as dinitro(1-methylheptyl)phenyl crotonate, 2-sec-butyl-4,6-dinitrophenyl 3,3-dimethylacrylate, 2-sec-butyl-4,6-dinitrophenylisopropyl carbonate, diisopropyl 5-nitro-isophthalate;
heterocyclic substances, such as 2-heptadecyl-2-imidazoline acetate, 2,4-dichloro-6-(o-chloroanilino)-s-triazine, O,O-diethyl phthalimidophosphonothioate, 5-amino-1-[bis(dimethylamino)phosphinyl]-3-phenyl-1,2,4- triazole, 2,3-dicyano-1,4-dithioanthraquinone, 2-thio-1,3-dithiolo[4,5-b]quinoxaline, methyl 1-(butylcarbamoyl)-2-benzimidazolecarbamate, 2-methoxycarbonylaminobenzimidazole, 2-(2-furyl)benzimidazole, 2-(4-thiazolyl)benzimidazole, N-(1,1,2,2-tetrachloroethylthio)tetrahydrophthalimide, N-trichloromethylthiotetrahydrophthalimide, N-trichloromethylthiophthalimide, N-dichlorofluoromethylthio-N',N'-dimethyl-N-phenylsulfo- diamide, 5-ethoxy-3-trichloromethyl-1,2,3-thiadiazole, 2-thiocyanatomethylthiobenzothiazole, 1,4-dichloro-2,5-dimethoxybenzene, 4-(2-chlorophenylhydrazono)-3-methyl-5-isoxazolone, pyridine-2-thiol 1-oxide, 8-hydroxyquinoline or its copper salt, 2,3-dihydro-5-carboxanilido-6-methyl-1,4-oxathiine, 2,3-dihydro-5-carboxanilido-6-methyl-1,4-oxathiine 4,4-dioxide, 2-methyl-5,6-dihydro-4H-pyran-3-carboxanilide, 2-methylfuran-3-carboxanilide, 2,5-dimethylfuran-3-carboxanilide, 2,4,5-trimethylfuran-3-carboxanilide, N-cyclohexyl- 2,5-dimethylfuran-3-carboxamide, N-cyclohexyl-N-methoxy-2,5-dimethylfuran-3-carboxamide, 2-methylbenzanilide, 2-iodobenzanilide, N-formyl-N-morpholine-2,2,2-trichloroethyl acetal, piperazine-1,4-diylbis-1-(2,2,2-trichioroethyl)formamide, 1-(3,4-dichloroanilino)-1-formylamino-2,2,2-trichloroethane; 2,6-dimethyl-N-tridecylmorpholine or its salts, 2,6-dimethyl-N-cyclododecylmorpholine or its salts, N-[3-(p-tert-butylphenyl)-2-methylpropyl]-cis-2,6-dimethyl- morpholine, N-[3-(p-tert-butylphenyl)-2-methylpropyl]-piperidine, 1-[2-(2,4-dichlorophenyl)-4-ethyl-1,3-dioxolan-2-yl-ethyl]-1H-1,2,4-triazole, 1-[2-(2,4-dichlorophenyl)-4-n-propyl-1,3-dioxolan-2-yl-ethyl]-1H-1,2,4-triazole, N-(n-propyl)-N-(2,4,6-trichlorophenoxyethyl)-N'-imidazolyl-urea, 1-(4-chlorophenoxy)-3,3-dimethyl-1-(1H-1,2,4-triazol-1-yl)-2-butanone, 1-(4-chlorophenoxy)-3,3-dimethyl-1-(1H-1,2,4-triaxol-1-yl)-2-butanol, (2RS,3RS)-1-[3-(2-chlorophenyl)-2-(4-fluorophenyl)-oxiran-2-ylmethyl]-1H-1,2,4-triazole, α-(2-chlorophenyl)-α-(4-chlorophenyl)-5-pyrimidinemethanol, 5-butyl-2-dimethylamino-4-hydroxy-6-methylpyrimidine, bis(p-chlorophenyl)-3-pyridinemethanol, 1,2-bis(3-ethoxycarbonyl-2-thioureido)benzene, 1,2-bis(3-methoxycarbonyl-2-thioureido)benzene, strobilurines such as azoxystrobin, kresoxim methyl, methyl-E-methoxyimino-[α-(2-phenoxyphenyl)]-acetamide, methyl E-methoxyimino-[α-(2,5-dimethylphenoxy)-o-tolyl]acetamide, picoxystrobin, pyraclostrobin, trifloxystrobin, anilinopyrimidines such as N-(4,6-dimethylpyrimidin-2-yl)aniline, N-[4-methyl-6-(1-propynyl)pyrimidin-2-yl]-aniline, N-[4-methyl-6-cyclopropylpyrimidin-2-yl]aniline, phenylpyrroles such as 4-(2,2-difluoro-1,3-benzodioxol-4-yl)pyrrole-3-carbonitrile, cinnamamides such as 3-(4-chlorophenyl)-3-(3,4-dimethoxyphenyl)acryloylmorpholine, and a variety of fungicides such as dodecylguanidine acetate, 3-[3-(3,5-dimethyl-2-oxycyclohexyl)-2-hydroxyethyl]glutarimide, hexachlorobenzene, methyl N-(2,6-dimethylphenyl)-N-(2-furoyl)-DL-alaninate, DL-N-(2,6-dimethylphenyl)-N-(2'-methoxyacetyl)-alanine methyl ester, N-(2,6-dimethylphenyl)-N-chloroacetyl-D,L-2-amino- butyrolactone, DL-N-(2,6-dimethylphenyl)-N-(phenylacetyl)alanine methyl ester, 5-methyl-5-vinyl-3-(3,5-dichlorophenyl)-2,4-dioxo-1,3-oxazolidine, 3-[3,5-dichlorophenyl(5-methyl-5-methoxymethyl]-1,3-oxazolidine-2,4-dione, 3-(3,5-dichlorophenyl)-l-isopropylcarbamoylhydantoin, N-(3,5-dichlorophenyl)-1,2-dimethylcyclopropane-1,2-dicarboximide, 2-cyano-[N-(ethylaminocarbonyl)-2-methoximino]acetamide, 1-[2-(2,4-dichloro-phenyl)pentyl]-1H-1,2,4-triazole, 2,4-difluoro-α-(1H-1,2,4-triazolyl-1-methyl)benzhydryl alcohol, N-(3-chloro-2,6-dinitro-4-trifluoromethylphenyl)-5-trifluoromethyl-3-chloro-2-aminopyridine, 1-((bis(4-fluorophenyl)methylsilyl)methyl)-1H-1,2,4-triazole.

### Synthesis Examples

With due modification of the starting compounds, the protocols shown in the synthesis examples below were used for obtaining further compounds I. The resulting compounds, together with physical data, are listed in the Table which follows.

### Example 1: Preparation of 5-chloro-7-hydroxy-6-(2-chloro-6-fluorophenyl)-[1,2,4]-triazolo[1,5-α]pyrimidine

To a solution of 32 g (0.1 mol) 5,7-dichloro-6-(2-chloro-6-fluorophenyl)-[1,2,4]-triazolo[1,5-α]pyrimidine [cf. EP-A 550 113] in 200 ml tetrahydrofuran (THF) 50 ml of a 10% aq. solution of sodium hydroxide was added with stirring. After 1 h additional 25 ml sodium hydroxide solution are added and stirring was continued for 1 h after which no more starting material could be detected by TLC. After 16 ml conc. hydrochloric acid were added volatiles were distilled off. The product was ectracted from the remainder with ethyl acetate. The two phase mixture was separated, the organic layer was dried. Adding light petroleum to the organic phase precipitated 22 g of the title compound as a tan powder of m.p. 257-259°C.

### Example 2: Preparation of 5-(4-methylpiperid-1-yl)-7-hydroxy-6-(2-chloro-6-fluorophenyl)-[1,2,4]-triazolo[1,5-α]pyrimidine as 4-methylpiperidinium salt

6.0 g (20 mmol) the compound from Example 1 and 12.0 g (120 mmol) 4-methylpiperidin were mixed and heated to 130°C for 4 days. After cooling to 50 to 60°C ethyl acetate was added and the precipitate formed was filtered off. 6.9 g of title compound were obtained after washing with ethyl acetate and drying.

### Example 3: Preparation of 5-(4-methylpiperid-1-yl)-7-chloro-6-(2-chloro-6-fluorophenyl)-[1,2,4]-triazolo[1,5-α]pyrimidine

A mixture of 3.6 g (7.5 mmol) Example 2 product and 15 ml phosphorous oxychloride was heated to 100°C for 4 hours. After cooling the reaction mixture was poured onto a mixture of ice and sodium carbonate. Extraction with toluene and chromatography on silica gel (toluene/ethyl acetate mixtures 5:1 to 2:1) yielded 2.9 g of the title compound of m.p. 137-140°C.

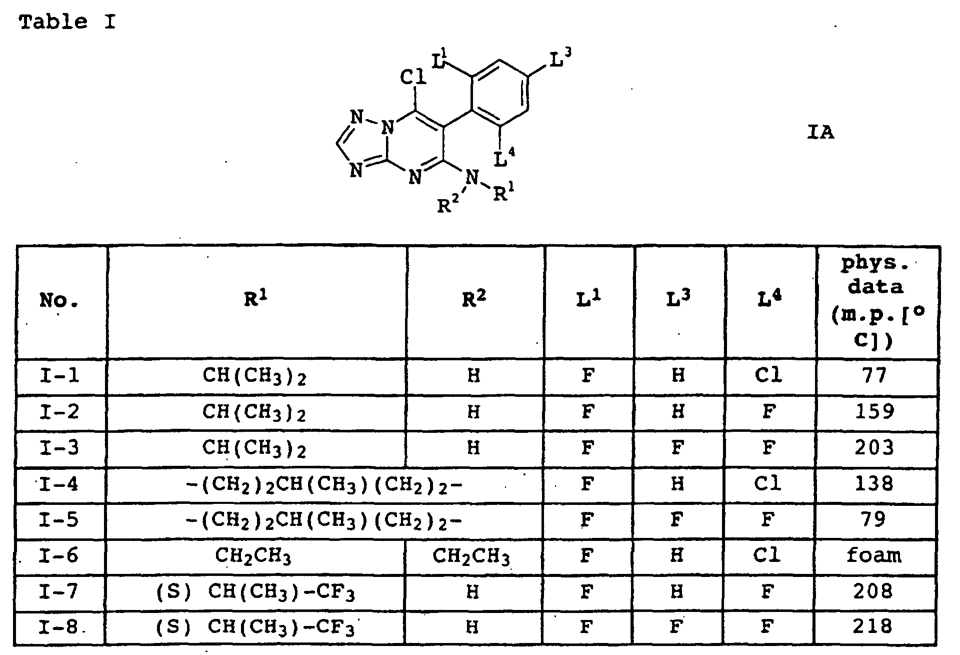

### Examples of the action against harmful fungi

The fungicidal action of the compounds of the formula I was demonstrated by the following experiments:

The active compounds, separately or together, were formulated as a 10% emulsion in a mixture of 70% by weight of cyclohexanone, 20% by weight of Nekanil® LN (Lutensol® AP6, wetting agent having emulsifying and dispersant action based on ethoxylated alkylphenols) and 10% by weight of Wettol® EM (nonionic emulsifier based on ethoxylated castor oil) and diluted with water to the desired concentration.

### Use example 1: Fungicidal control of apple scab

### (Venturia inequalis)

Young apple seedlings of the cultivar "Common" were grown in pots to the 4 to 5 leaf stage. These plants were sprayed to run-off with an aqueous suspension, containing the concentration of active ingredient mentioned in the table below, prepared from a stock solution containing 5 % of the active ingredient, 94 % cyclohexanone and 1 % emulsifier (Tween 20). After the plants had dried (3-5h), they were inoculated with an aqueous spore suspension of Venturia inequalis. Then the trial plants were immediately transferred to a humid chamber with 22 to 24°C and a relative humidity close to 100 % and were cultivated there for 2 days. For a period of further 2 weeks a cultivation in a greenhouse followed at 21 to 23°C and a relative humidity about 95 %. Then the extent of fungal attack on the leaves was visually assessed as % diseased leaf area.

In this test, the plants which had been treated with 12.5 ppm of the compound I-4 showed an infection of 7%, whereas the untreated plants were infected to 80%.

### Use example 2: Fungicidal control of early blight on tomatoes (Alternaria solani)

Young tomato seedlings of the cultivar "Pixie II" were grown in pots to the 2 to 4 leaf stage. These plants were sprayed to run-off with an aqueous suspension, containing the concentration of active ingredient mentioned in the table below, prepared from a stock solution containing 5 % of the active ingredient, 94 % cyclohexanone and 1 % emulsifier (Tween 20).

After the plants had dried (3-5h), they were inoculated with an aqueous spore suspension of Alternaria solani containing 15 x 10³ spores per ml. Then the trial plants were immediately transferred to a humid chamber with 22 to 24°C and a relative humidity close to 100 % for 36 h. For a period of further 2 to 3 days a cultivation in a greenhouse followed at 21 to 23°C and a relative humidity about 95 %. Then the extent of fungal attack on the leaves was visually assessed as % diseased leaf area.

In this test, the plants which had been treated with 50 ppm of the compound I-4 showed an infection of 3%, whereas the untreated plants were infected to 90%.

### Use example 3: Fungicidal control of leaf spot on beets (Cercospora beticola)

Young sugar beet seedlings of the cultivar "ACH-31" were grown in pots to the 2 to 4 leaf stage. These plants were sprayed to run-off with an aqueous suspension, containing the concentration of active ingredient mentioned in the table below, prepared from a stock solution containing 5 % of the active ingredient, 94 % cyclohexanone and 1 % emulsifier (Tween 20). After the plants had dried (3-5h), they were inoculated with a spore suspension of Cercospora beticola in an aqueous solution of 0.5 % gelatine. Then the trial plants were immediately transferred to a humid chamber with 18 to 23°C and a relative humidity close to 100 % and kept there for 5 days. For a period of further 10 to 14 days a cultivation in a greenhouse followed at 21 to 23°C and a relative humidity about 95 %. Then the extent of fungal attack on the leaves was visually assessed as % diseased leaf area.

In this test, the plants which had been treated with 50 ppm of the compound I-4 showed an infection of 7%, whereas the untreated plants were infected to 70%.

## Claims

1. 5-Alkylamino-6-phenyl-7-halo-triazolopyrimidines of formula I 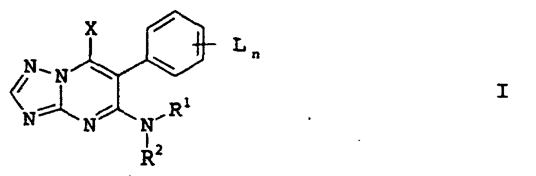 in which
R¹ and R² independently denote hydrogen or
C₁-C₁₀-alkyl, C₂-C₁₀-alkenyl, C₂-C₁₀-alkynyl, C₄-C₁₀-alkadienyl, or C₁-C₁₀-haloalkyl,
C₃-C₈-cycloalkyl, C₅-C₁₀-bicycloalkyl, phenyl, naphthyl, or
5- or 6-membered heterocyclyl, containing one to four nitrogen atoms or one to three nitrogen atoms and one sulfur or oxygen atom, or
5- or 6-membered heteroaryl, containing one to four nitrogen atoms or one to three nitrogen atoms and one sulfur or oxygen atom, or
where R¹ and R² radicals may be unsubstituted or partially or fully halogenated or may carry one to three groups R^{a},
R^{a} is halogen, cyano, nitro, hydroxyl, C₁-C₆-alkyl, C₁-C₆-haloalkyl, C₃-C₆-cycloalkyl, C₁-C₆-alkoxy, C₁-C₆-haloalkoxy, C₁-C₆-alkylthio, C₁-C₆-alkylamino, di-C₁-C₆-alkylamino, C₂-C₆-alkenyl, C₂-C₆-alkenyloxy, C₂-C₆-alkynyl, C₃-C₆-alkynyloxy and C₁-C₄-alkylenedioxy, which may be halogenated; or
R¹ and R² together with the interjacent nitrogen atom represent a 5- or 6-membered heterocyclic ring, containing one to four nitrogen atoms or one to three nitrogen atoms and one sulfur or oxygen atom, which may be substituted by one to three R^{a} radicals;
L is hydrogen, halogen, C₁-C₁₀ alkyl, C₁-C₁₀ alkoxy and C₁-C₁₀ haloalkyl;
n is an integer from 1 to 5; and
X is halogen.

2. Compounds of formula I according to claim 1, in which
R¹ is straight chained or branched C₁-C₆-alkyl, C₁-C₆-haloalkyl, straight chained or branched C₂-C₆-alkenyl, C₃-C₆-cycloalkyl, or C₅-C₉-bicycloalkyl, and
R² is hydrogen or C₁-C₆-alkyl, or
R¹ and R² together with the interjacent nitrogen atom represent a heterocyclic ring with 5 or 6 carbon atoms being optionally substituted with one or two C₁-C₆-alkyl groups;
Lₙ is 2-chloro-6-fluoro, 2,6-difluoro, 2,6-dichloro, 2-methyl-6-fluoro, 2,4,6-trifluoro, 2,6-difluoro-4-methoxy, or pentafluoro.

3. A process for the preparation of compounds of formula I as defined in claims 1 and 2 which comprises reacting 5,7-dihalo-6-phenyl-triazolopyrimidines of formula II 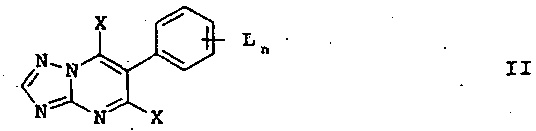 in which X is halogen with a base to 7-hydroxycompounds of formula III 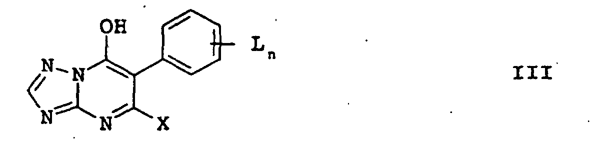 in which X is halogen and after neutralisation reacting with an amine of formula IV 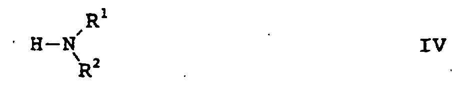 in which R¹ and R² are defined as in formula I to give 5-amino-7-hydroxycompounds of formula V 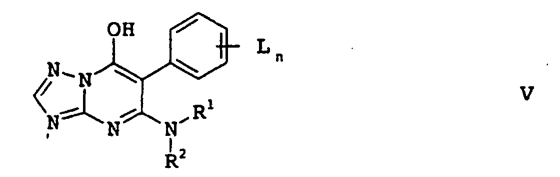 and further halogenation to produce compounds of formula I.

4. Compounds of formulae III and V as defined in claim 3.

5. A composition suitable for controlling phytopathogenic fungi, comprising a solid or liquid carrier and a compound of the formula I as claimed in claim 1.

6. A method for controlling phytopathogenic fungi, which comprises treating the fungi or the materials, plants, the soil or the seed to be protected against fungal attack with an effective amount of a compound of the formula I as claimed in claim 1.

## Patentansprüche

1. 5-Alkylamino-6-phenyl-7-halogentriazolopyrimidine der Formel I, 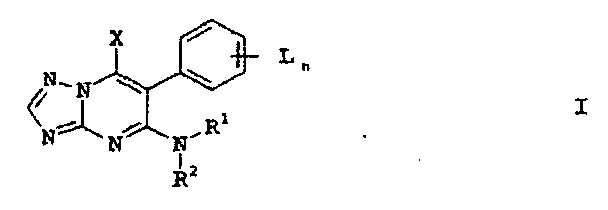 in der
R¹ und R² unabhängig Wasserstoff oder
C₁-C₁₀-Alkyl, C₂-C₁₀-Alkenyl, C₂-C₁₀-Alkinyl, C₄-C₁₀-Alkadienyl oder C₁-C₁₀-Halogenalkyl,
C₃-C₈-Cycloalkyl, C₅-C₁₀-Bicycloalkyl, Phenyl, Naphthyl oder 5- oder 6-gliedriges Heterocyclyl, das ein bis vier Stickstoffatome oder ein bis drei Stickstoffatome und ein Schwefel- oder Sauerstoffatom enthält, oder
5- oder 6-gliedriges Heteroaryl, das ein bis vier Stickstoffatome oder ein bis drei Stickstoffatome und ein Schwefel- oder Sauerstoffatom enthält,
bedeuten, oder
wo die R¹- und R²-Reste unsubstituiert oder teilweise oder ganz halogeniert sein können oder eine bis drei Gruppen R^{a},
wobei R^{a} Halogen, Cyano, Nitro, Hydroxy, C₁-C₆-Alkyl, C₁-C₆-Halogenalkyl, C₃-C₆-Cycloalkyl, C₁-C₆-Alkoxy, C₁C₆-Halogenalkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkinyl, C₃-C₆-Alkinyloxy und C₁-C₄-Alkylendioxy, das halogeniert sein kann, bedeutet,
tragen können, oder
R¹ und R² gemeinsam mit dem dazwischenliegenden Stickstoffatom einen 5- oder 6-gliedrigen Heterocyclus, der ein bis vier Stickstoffatome oder ein bis drei Stickstoffatome und ein Schwefel- oder Sauerstoffatom enthält und der durch ein bis drei R^{a}-Reste substituiert sein kann, bedeuten;
L Wasserstoff, Halogen, C₁-C₁₀-Alkyl, C₁-C₁₀-Alkoxy und C₁-C₁₀-Halogenalkyl bedeutet;
n eine ganze Zahl von 1 bis 5 bedeutet; und
X Halogen bedeutet.

2. Verbindungen der Formel I nach Anspruch 1, in der
R¹ geradkettiges oder verzweigtes C₁-C₆-Alkyl, C₁-C₆-Halogenalkyl, geradkettiges oder verzweigtes C₂-C₆-Alkenyl, C₃-C₆-Cycloalkyl oder C₅-C₉-Bicycloalkyl bedeutet, und
R² Wasserstoff oder C₁-C₆-Alkyl bedeutet, oder
R¹ und R² gemeinsam mit dem dazwischenliegenden Stickstoffatom einen Heterocyclus mit 5 oder 6 Kohlenstoffatomen, der gegebenenfalls durch eine oder zwei C₁-C₆-Alkylgruppen substituiert ist, bedeuten;
Lₙ 2-Chlor-6-fluor, 2,6-Difluor, 2,6-Dichlor, 2-Methyl-6-fluor, 2,4,6-Trifluor, 2,6-Difluor-4-methoxy oder Pentafluor bedeutet.

3. Verfahren zur Herstellung von Verbindungen der Formel I nach Anspruch 1 und 2, das **dadurch gekennzeichnet ist, daß** man 5,7-Dihalogen-6-phenyltriazolopyrimidine der Formel II, 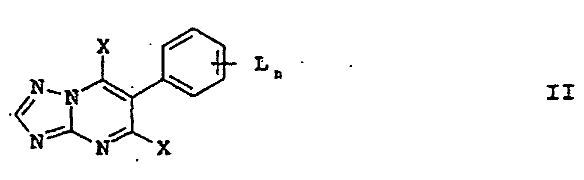 in der X Halogen bedeutet, mit einer Base zu 7-Hydroxy-Verbindungen der Formel III, 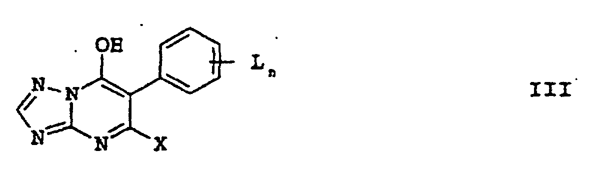 in der X Halogen bedeutet, umsetzt und das Produkt nach dem Neutralisieren mit einem Amin der Formel IV, 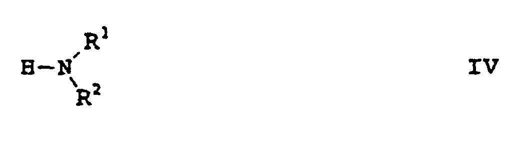 in der R¹ und R² wie in Formel I definiert sind, zu 5-Amino-7-hydroxy-Verbindungen der Formel V 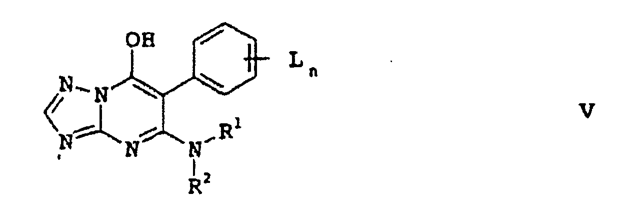 umsetzt und weiter zu Verbindungen der Formel I halogeniert.

4. Verbindungen der Formel III und V nach Anspruch 3.

5. Zusammensetzung, die sich für die Bekämpfung von phytopathogenen Pilzen eignet, umfassend einen festen oder flüssigen Träger und eine Verbindung der Formel I nach Anspruch 1.

6. Verfahren zur Bekämpfung von phytopathogenen Pilzen, **dadurch gekennzeichnet, daß** man die Pilze oder die Substanzen, Pflanzen, den Boden oder das Saatgut, die, der bzw. das gegen Pilzbefall zu schützen ist, mit einer wirksamen Menge einer Verbindung der Formel I nach Anspruch 1 behandelt.

## Revendications

1. 5-alkylamino-6-phényl-7-halogénotriazolopyrimidines de formule I 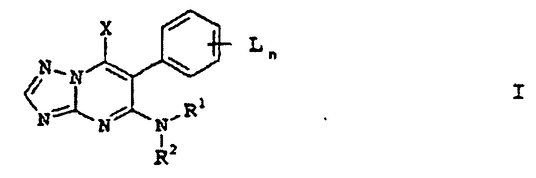 dans laquelle
R¹ et R² représentent indépendamment un atome d'hydrogène ou
un groupe alkyle en C₁ à C₁₀, un groupe alcényle en C₂ à C₁₀, un groupe alcynyle en C₂ à C₁₀, un groupe alcanediényle en C₄ à C₁₀ ou un groupe halogénoalkyle en C₁ à C₁₀,
un groupe cycloalkyle en C₃ à C₈, un groupe bicycloalkyle en C₅ à C₁₀, un groupe phényle, un groupe naphtyle, ou
un groupe hétérocyclyle comprenant 5 à 6 membres, contenant un à quatre atomes d'azote ou un à trois atomes d'azote et un atome de soufre ou atome d'oxygène, ou
un groupe hétéroaryle comprenant 5 à 6 membres, contenant un à quatre atomes d'azote ou un à trois atomes d'azote et un atome de soufre ou atome d'oxygène, ou
les radicaux R¹ et R² peuvent être non substitués ou partiellement ou complètement halogénés ou peuvent porter un à trois groupes R^{a},
R^{a} représentant un atome d'halogène, un groupe cyano, un groupe nitro, un groupe hydroxyle, un groupe alkyle en C₁ à C₆, un groupe halogénoalkyle en C₁ à C₆, un groupe cycloalkyle en C₃ à C₆, un groupe alcoxy en C₁ à C₆, un groupe halogénoalcoxy en C₁ à C₆, un groupe alkylthio en C₁ à C₆, un groupe (alkyle en C₁ à C₆)amino, un groupe di-(alkyle en C₁ à C₆)amino, un groupe alcényle en C₂ à C₆, un groupe (alcényle en C₂ à C₆)oxy, un groupe alcynyle en C₂ à C₆, un groupe (alcynyle en C₃ à C₆)oxy et un groupe (alkylène en C₁ à C₄)dioxy, qui peut être halogéné ; ou
R¹ et R² forment ensemble avec l'atome d'azote adjacent intermédiaire un hétérocycle comprenant 5 à 6 membres, contenant un à quatre atomes d'azote ou un à trois atomes d'azote et un atome de soufre ou atome d'oxygène, qui peut être substitué par un à trois radicaux R^{a} ;
L représente un atome d'hydrogène, un atome d'halogène, un groupe alkyle en C₁ à C₁₀, un groupe alcoxy en C₁ à C₁₀ et un groupe halogénoalkyle en C₁ à C₁₀ ;
n représente un entier de 1 à 5 ; et
X représente un atome d'halogène.

2. Composés de formule 1 selon la revendication 1, dans laquelle
R¹ est un groupe alkyle en C₁ à C₆ à chaîne linéaire ou ramifiée, un groupe halogénoalkyle en C₁ à C₆, un groupe alcényle en C₂ à C₆ à chaîne linéaire ou ramifiée, un groupe cycloalkyle en C₃ à C₆ ou un groupe bicycloalkyle en C₅ à C₉, et
R² représente un atome d'hydrogène ou un groupe alkyle en C₁ à C₆, ou
R¹ et R² forment ensemble avec l'atome d'azote adjacent intermédiaire un hétérocycle comprenant 5 ou 6 atomes de carbone, éventuellement substitué par un ou deux groupes alkyle en C₁ à C₆ ;
Lₙ représente un groupe 2-chloro-6-fluoro, 2,6-difluoro, 2,6-dichloro, 2-méthyl-6-fluoro, 2,4,6-trifluoro, 2,6-difluoro-4-méthoxy ou pentafluoro.

3. Procédé pour la préparation de composés de formule I telle que définie dans les revendications 1 et 2, comprenant la réaction de 5,7-dihalogéno-6-phényltriazolopyrimidines de formule II 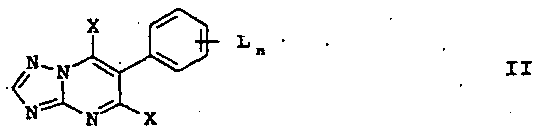 dans laquelle X représente un atome d'halogène avec une base en composés 7-hydroxy de formule III 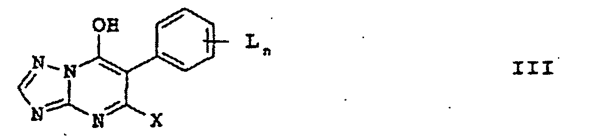 dans laquelle X représente un atome d'halogène et, après la neutralisation, la réaction avec une amine de formule IV 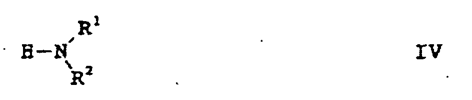 dans laquelle R¹ et R² sont tels que définis dans la formule I pour obtenir les composés 5-amino-7-hydroxy de formule V 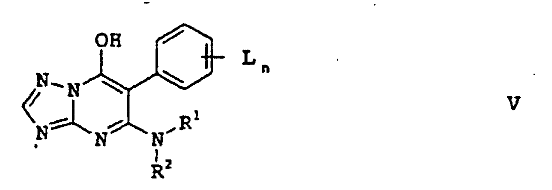 puis une halogénation pour produire les composés de formule I.

4. Composés de formules III et V telles que définies dans la revendication 3.

5. Composition appropriée pour le contrôle des champignons phytopathogènes, comprenant un support solide ou liquide et un composé de formule I telle que définie dans la revendication 1.

6. Procédé pour le contrôle de champignons phytopathogènes, comprenant le traitement des champignons ou des matériaux, des plantes, du sol ou des graines à protéger contre une attaque par les champignons avec une quantité efficace d'un composé de formule 1 telle que définie dans la revendication 1.
